# EUROPEAN PATENT APPLICATION

(11) **EP 3 979 257 A1**
(43) Date of publication of application: **06.04.2022**
(21) Application number: 21199736.6
(22) Date of filing: 29.09.2021
(51) Int. Cl.: G16H 50/20

(54) **ENABLING UTILIZATION OF MASSIVE DATA IN A SERVICE**

(30) Priority: 30.09.2020 FI 20205945
(71) Applicant: Avaintec Oy, 00180 Helsinki (FI)
(72) Inventor: Valmari, Pekka, 00180 Helsinki (FI); Tabatabaei, Mohammad, 00180 Helsinki (FI); Kuosmanen, Pekka, 00180 Helsinki (FI)
(74) Representative: Kolster Oy Ab

(57) **Abstract**

A method, comprising: receiving input data from a network connection between two devices, providing the input data to a determination unit, labelling the input data using the determination unit, wherein the determination unit utilizes a plurality of Machine Learning algorithms that has been trained on a database, and wherein labelling comprises obtaining labelling likelihoods from a plurality of Machine Learning algorithms, and providing an indication regarding the labelled input data.

## Description

### FIELD

The present application relates to extracting value from large amounts of data with the help of artificial intelligence and machine learning.

### BACKGROUND

If a user, who may be a professional or who may need to seek for professional advice needs to act on insufficient information, that may easily result in, for example, jammed helplines of a service provider, inferior decisions being made by the user, or even casualties being caused due to decisions made based on lacking knowledge. Bringing knowledge stored in large databases of information widely and readily available to users would therefore increase the ability of the users to make more informed decisions, which may benefit many parties.

### BRIEF DESCRIPTION

The scope of protection sought for various embodiments of the invention is set out by the independent claims. The exemplary embodiments and features, if any, described in this specification that do not fall under the scope of the independent claims are to be interpreted as examples useful for understanding various embodiments of the invention.

According to one aspect there is provided an apparatus comprising means for receiving input data from a network connection between two devices, providing the input data to a determination unit, labelling the input data using the determination unit, wherein the determination unit utilizes a plurality of Machine Learning algorithms that has been trained on a database, and wherein labelling comprises obtaining labelling likelihoods from a plurality of Machine Learning algorithms, and providing an indication regarding the labelled input data.

According to another aspect there is provided an apparatus comprising at least one processor, and at least one memory including a computer program code, wherein the at least one memory and the computer program code are configured, with the at least one processor, to cause the apparatus to receive input data from a network connection between two devices, provide the input data to a determination unit, label the input data using the determination unit, wherein the determination unit utilizes a plurality of Machine Learning algorithms that has been trained on a database, and wherein labelling comprises obtaining labelling likelihoods from a plurality of Machine Learning algorithms, and provide an indication regarding the labelled input data.

According to another aspect there is provided a method comprising receiving input data from a network connection between two devices, providing the input data to a determination unit, labelling the input data using the determination unit, wherein the determination unit utilizes a plurality of Machine Learning algorithms that has been trained on a database, and wherein labelling comprises obtaining labelling likelihoods from a plurality of Machine Learning algorithms, and providing an indication regarding the labelled input data.

According to another aspect there is provided a computer program product readable by a computer and, when executed by the computer, configured to cause the computer to execute a computer process comprising receiving input data from a network connection between two devices, providing the input data to a determination unit, labelling the input data using the determination unit, wherein the determination unit utilizes a plurality of Machine Learning algorithms that has been trained on a database, and wherein labelling comprises obtaining labelling likelihoods from a plurality of Machine Learning algorithms, and providing an indication regarding the labelled input data.

According to another aspect there is provided a computer program product comprising computer-readable medium bearing computer program code embodied therein for use with a computer, the computer program code comprising code for performing receiving input data from a network connection between two devices, providing the input data to a determination unit, labelling the input data using the determination unit, wherein the determination unit utilizes a plurality of Machine Learning algorithms that has been trained on a database, and wherein labelling comprises obtaining labelling likelihoods from a plurality of Machine Learning algorithms, and providing an indication regarding the labelled input data.

According to another aspect there is provided a computer program comprising instructions for causing an apparatus to perform at least the following: receiving input data from a network connection between two devices, providing the input data to a determination unit, labelling the input data using the determination unit, wherein the determination unit utilizes a plurality of Machine Learning algorithms that has been trained on a database, and wherein labelling comprises obtaining labelling likelihoods from a plurality of Machine Learning algorithms, and providing an indication regarding the labelled input data.

According to another aspect there is provided a computer-readable medium comprising program instructions for causing an apparatus to perform at least the following: receiving input data from a network connection between two devices, providing the input data to a determination unit, labelling the input data using the determination unit, wherein the determination unit utilizes a plurality of Machine Learning algorithms that has been trained on a database, and wherein labelling comprises obtaining labelling likelihoods from a plurality of Machine Learning algorithms, and providing an indication regarding the labelled input data.

According to another aspect there is provided a non-transitory computer-readable medium comprising program instructions for causing an apparatus to perform at least the following: receiving input data from a network connection between two devices, providing the input data to a determination unit, labelling the input data using the determination unit, wherein the determination unit utilizes a plurality of Machine Learning algorithms that has been trained on a database, and wherein labelling comprises obtaining labelling likelihoods from a plurality of Machine Learning algorithms, and providing an indication regarding the labelled input data.

### BRIEF DESCRIPTION OF THE DRAWINGS

Figures 1a and 1b illustrate two exemplary embodiments of environments.
Figure 2 illustrates an exemplary embodiment of a system extracting value from the information comprised in a large database.
Figures 3-6 illustrate flow charts according to exemplary embodiments.
Figure 7 illustrates an exemplary embodiment of an apparatus.

### DETAILED DESCRIPTION

One person, even an expert, has limited resources to resolve a problematic situation. There may exist a helpline or another external resource to contact, but even that expertise has its limits. A system bringing the information and expertise of a large database available remotely to an individual user, for example in an urgent situation, is therefore beneficial. The database may comprise information collected from many resources and over various time-periods and may therefore hold more knowledge than an individual or a group of individuals working together. One example of such system is a service available for patients or caretakers, by which they may check the need for visiting a hospital emergency room in a case of illness, by a network connection without a load to helpline personnel. Another example is a service for a process controller for determining, for example, if a malfunction in the process may be resolved without a pause in the process. Such systems may for example deploy artificial intelligence (AI) to exploit so large amount of data that it would exceed the capacity of any one expert. The user may then connect remotely to the service using his device and Internet connection for example. Suchlike service may be suited also to other applications profiting from extracting value from a large database remotely by anyone.

Artificial intelligence may be defined as a computer system performing tasks that ordinarily require human intelligence. A computer system may comprise one or a plurality of computers, one or a plurality of remote devices, and/or a cloud, or any combination thereof. Such tasks may comprise, for example, analysing the performance and efficacy of manufacturing equipment in a factory, analysing sales calls to improve marketing strategies, or inspecting public transport usage to economise. Artificial intelligence may process enormous quantities of data in a short time, for example, healthcare data of a hospital district or a public transport system database of a municipality. Processing these may be an impossible task to a human or a group of humans. Artificial intelligence may detect connections and impacts in data that may escape human intelligence. These attributes give artificial intelligence an advantage compared to human intelligence.

Machine learning (ML) may be understood as an application of artificial intelligence that provides a computer system the ability to automatically learn and improve from experience without being explicitly programmed. Machine learning may focus on the development of computer programs that may access data and use it to learn for themselves. An algorithm is a description of the steps of a method. Thus, a machine learning algorithm comprises the steps for a machine learning method for producing a model that can then be utilized to derive conclusions on new data. In this context the term data is used for any units of information.

Input data is data that may be provided as an input to a device or an algorithm. The input data for a machine learning algorithm comprises individual measurable characteristics of a phenomenon being observed, which may be called features. In this context these features are called data features, to distinguish them from other possible features. Data features may comprise, for example, service intervals for machinery, wear of equipment in different circumstances in a factory, visits to the hospital and laboratory test results in a hospital database, or numbers of passengers on various bus routes recorded on a database of a public transport system. To be usable as input data for machine learning algorithms, input data may be organized as an array or vector of numbers. Therefore, non-numerical input data is converted to numbers, for example by dividing different options of a characteristic as separate data features having binary values according to their realization. This may be implemented, for example, by applying one-hot encoding.

Machine learning methods may be categorized broadly as supervised or unsupervised. Supervised methods may apply what has been learned in the past using labelled examples to predict future events. A supervised algorithm may require a set of data known as training data, comprising input data and labels that are considered as the output values of machine learning algorithms. Starting from the analysis of the training data, the machine learning algorithm produces a model, such as a mathematical model, that makes predictions about the output values. After sufficient training, the model may provide accurate output values for any new input data. The accuracy of the model, that is, the adequacy of the training may be validated by techniques such as cross-validation method. In the method some part of the labelled training data is not used for training but saved for estimating the model accuracy, that is, if the model provides a correct label to an input data that already has a known output value. An unsupervised algorithm in turn takes a set of data that comprises input data without labels and finds a structure in the input data, like grouping or clustering of data points. Therefore, unsupervised methods do not need a labelled input data for training in contrast to supervised methods but may learn from input data that has not been labelled.

There are numerous options for the mathematical model to be applied in a machine learning method, and the choice may depend on the scope of the input data and desired output values. Such models comprise, for example, logistic regression model, support vector machine model, random forest model, and naive Bayes model. These models all use different constructions to fit into the input data. A machine learning algorithm, for example, chooses parameters for a mathematical formula or components for a tree structure, so that the resulting model best fits into the input data. Logistic regression model uses the logistic function, such as f(x) = 1 / (1 + e^{-x}), where e is the natural logarithm. Support vector machine model constructs a hyperplane or a set of hyperplanes in a high-dimensional space. Random forest model constructs a multitude of decision trees that are structures that go from observations about an item to conclusions about the item's target value. Naive Bayes model applies Bayes' theorem that describes the probability of an event based on prior knowledge of conditions that might be related to the event, with strong independence assumptions between data features. It is also possible to combine models to improve the performance of a method.

The results of a machine learning method may be provided as likelihoods for the possible output values, that is, labels. These likelihoods indicate the label or labels that may be the most probable output value or values for a provided input data. The magnitude of the likelihoods may be used to deduce the certainty of the predicted output value.

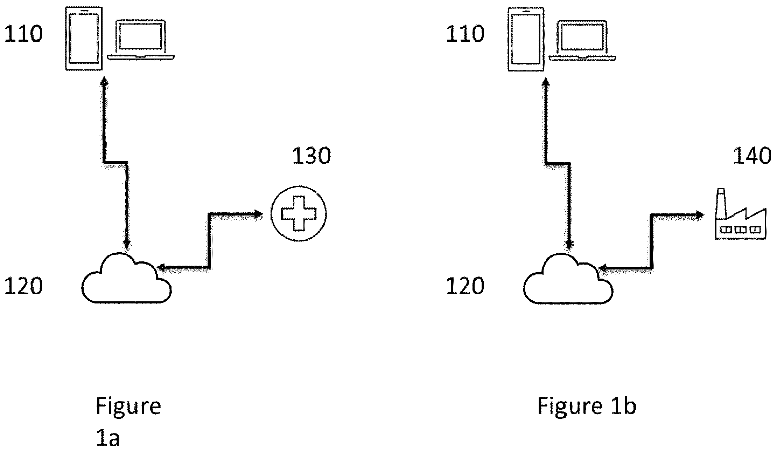

Figures 1a and 1b illustrate two exemplary embodiments of environments that would benefit from a use of large amount of data by the help of artificial intelligence and/or machine learning. In this context an environment is meant as a set-up comprising one or more user devices, one or more computing devices executing one or more algorithms, and/or one or more databases, or a combination thereof. A user device 110 may be for example a mobile phone, a personal computer, a tablet, or any other device capable of connecting to the Internet and optionally also installing applications. A cloud 120 may be a computational unit comprising one or more computing devices, a database, a computer unit, or a combination thereof, comprising storage and capable of performing cloud computing. A hospital or other healthcare unit 130, which may be for example a hospital, a doctor's office, a health centre, or other unit, illustrated in Figure 1a may store data to the cloud 120 and/or provide the cloud 120 an access to an internal database. The cloud 120 may get data from the user device 110 for processing and send the data further to the hospital 130. The cloud 120 may provide the results of the processing to the user device 110 and/or the hospital 130. This connection between the user device 110 and the hospital 130 via the cloud 120 may enable the user device 110 to send and/or receive information regarding, for example, the state of health of the person operating the user device 110, the person's need for an urgent hospital visit in case of an acute illness or an accident, or additional need for emergency service personnel or for example cellular blood products in the hospital 130. Further, the cloud computing 120 may comprise a database that comprises data from various sources and which may be utilized using artificial intelligence and/or machine learning to extract knowledge to be utilized by the user device 110 and/or the hospital 130.

A factory or other manufacturing unit 140 illustrated in Figure 1b may store data to the cloud 120 and/or provide the cloud 120 an access to an internal database. The cloud 120 may get data from the user device 110 for processing and send the data further to the factory 140. The cloud may provide the results of the processing to the user device 110 and/or the factory 140. This connection between the user device 110 and the factory 140 via the cloud 120 may enable the user device 110 to send and/or receive information regarding, for example, equipment performance and machinery condition, operational instructions for problem solving in case of an unexpected malfunction of equipment, or additional need of personnel or equipment in the factory 140. Further, the cloud computing 120 may comprise a database that comprises data from various sources and which may be utilized using artificial intelligence and/or machine learning to extract knowledge to be utilized by the user device 110 and/or the factory 140.

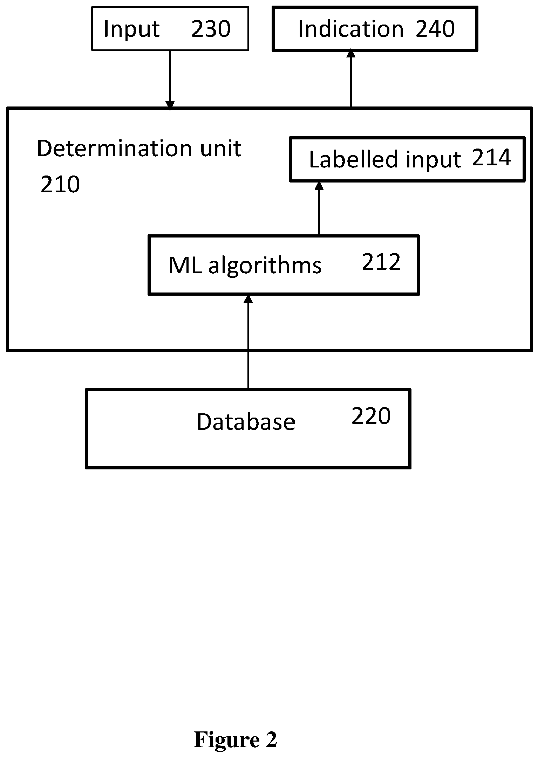

An exemplary embodiment of a system extracting value from the information comprised in a large database with the help of artificial intelligence and machine learning is illustrated in Figure 2. A determination unit 210 is a logical unit that may be located in a cloud or other computer device. The determination unit 210 comprises machine learning algorithms 212 that comprise one or a plurality of machine learning methods trained with data in a database 220. The database 220 may be located in a cloud or other computer device, which can be the same as the one comprising the determination unit 210, or a different cloud or other computer device. The database 220 may be connected to the determination unit 210 by a network connection. An input 230 may be given to the determination unit 210 by a user remotely, for example over an Internet connection. The input 230 may comprise for example audio files, log files, filled questionnaires or any other data available to a computing device, and the database 220 comprises other data, for example other audio files, log files, filled questionnaires or other data available to a computing device. In this context the input may be understood as input data usable by machine learning algorithms or some data that may be converted to an input data. In some exemplary embodiments, a user may have an application installed in a user device and the application may be utilized to provide the input 230. For example, the user may select to fill in a questionnaire provided by the application or the user may select one or more options provided by the application and the application then transmits the user input to the determination unit 210 as the input 230. The machine learning algorithms 212 may then label the input 230 thereby producing a labelled input 214. The determination unit 210 may then provide an indication 240 regarding the labelled input 214. The indication 240 may for example comprise operating instructions, warning signals, advice, or other guidance. In some exemplary embodiments, the indication 240 may be provided to the user device from which the input 230 was received, or, alternatively or additionally, it may be provided to any other suitable device as well. The indication 240 may then be rendered using audio, graphical user interface or any other suitable means or a combination thereof.

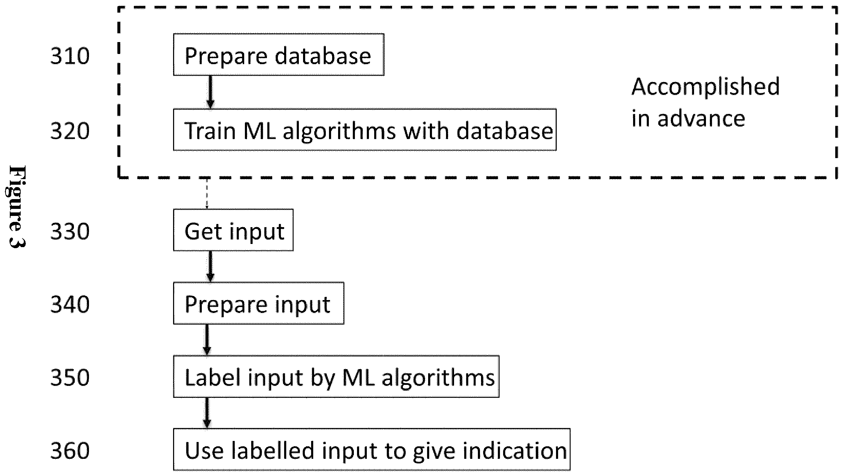

Figure 3 illustrates a flow chart according to an exemplary embodiment of training machine learning algorithms with a database and using the trained algorithms to label input. The training is accomplished when constructing the service but it may also be conducted again later on at any suitable time with, for example a larger or a later database to improve the performance of the environment extracting value from large amounts of data. Preparation of the database 310 comprises verifying that the format of the data is such that it is compatible with the machine learning algorithms or, additionally, or alternatively, converting the data to a format that is compatible with the machine learning algorithms. The format may for example be numbers. Training of machine learning algorithms with a database 320 comprises using the prepared database and one or a plurality of machine learning methods to obtain algorithms that will provide as results for any new input data labels comprised in the training data. For example, if the training data comprises five different labels, the machine learning algorithms may provide these five labels as output for an input. This may comprise using one or a plurality of machine learning methods and an algorithm to combine the results of the plurality of methods. The plurality of methods may comprise, for example, four or five, or any suitable number of machine learning methods and they may be supervised machine learning methods. The methods may exploit various models as introduced previously, such as logistic regression model, support vector machine model, random forest model, naive Bayes model, or other models. When the machine learning algorithms are trained and ready to be used for labelling, an input 330 may be obtained from a user for example using a network connection. The network connection may in some alternative exemplary embodiments be optional though. The network connection may be a connection to a user device in some exemplary embodiments. Preparation of the input 340 comprises verifying that the format of the input is such that it is compatible with the machine learning algorithms or, additionally, or alternatively, converting the input to a format that is compatible with the machine learning algorithms. The format may for example be numbers. Labelling the input by the trained machine learning algorithms 350 comprises obtaining likelihoods for possible labels by feeding the prepared input to the machine learning algorithms and getting the likelihoods as a result. The likelihoods may be obtained by one machine learning algorithm or a plurality of machine learning algorithms. If a plurality of machine learning algorithms is used, the label likelihoods are determined by all the machine learning algorithms in use independently. Then the label likelihoods for the plurality of machine learning algorithms may be determined by calculating averages of the label likelihoods for groups of labels. A group of labels may comprise one label or a plurality of labels. The averages may be obtained by calculating, for example, an arithmetic mean, a median, a mode, some other measure of central tendency, or by some other means. The arithmetic mean is the sum of a collection of numbers divided by the count of numbers in the collection. The median is the middle number of a list of numbers, when the numbers are listed in order from smallest to greatest. The mode is the value that appears most often in a set of data values.

In an exemplary embodiment there maybe four groups of labels. In each group there may be one possible label for an output value. There may be three machine learning algorithms in this exemplary embodiment. For a given input data, each machine learning algorithm may provide an individual likelihood of each group of labels for that input data. The machine learning algorithms provide the individual likelihoods without being influenced by the other machine learning algorithms utilized. As the individual likelihoods may be obtained individually from each machine learning algorithm first, then for each group of labels, an average of the individual likelihoods of that group may be obtained and provided as an output. In the output the likelihood of a group of labels is the average of the individual likelihoods obtained for that group by the individual machine learning algorithms. In another exemplary embodiment there may be three groups of labels, a first comprising three labels, a second comprising six labels and a third comprising one label. This division into groups may be done on the basis of some attributes of the labels, or on the basis of some other criteria. There may be four machine learning algorithms in this exemplary embodiment. For a given input data, a first and a second of the machine learning algorithms may provide likelihoods of all the groups of labels in this exemplary embodiment for that input data, a third machine learning algorithm may provide a likelihood of the first and the second groups of labels for that input data, and a fourth machine learning algorithm may provide likelihoods for the second and the third groups of labels for that input data. As the individual likelihoods for each group of labels may be obtained individually from one or more of the machine learning algorithms first, then for each group of labels, an average of the likelihoods of that group may be obtained and provided as an output. In the output the likelihood of a group of labels is the average of the individual likelihoods obtained for that group by the individual machine learning algorithms. The first group of labels may have individual likelihoods provided by the first, the second and the third of the machine learning algorithms in this exemplary embodiment, and the average may be obtained from these individual likelihoods. The second group of labels may have individual likelihoods provided by all the machine learning algorithms in this exemplary embodiment, and the average may be obtained from these individual likelihoods. The third group of labels may have individual likelihoods provided by the first, the second and the fourth machine learning algorithms in this exemplary embodiment, and the average may be obtained from these individual likelihoods.

An indication 360 may be given based on the label likelihoods, which may be the label likelihoods obtained from one machine learning algorithm or the averages calculated for the groups of labels from the label likelihoods obtained from the plurality of machine learning algorithms. If the likelihoods are determined for a group of labels, the group and/or the labels in the group may be indicated in the indication 360. In other words, the information of obtained likelihoods for possible labels for the input data may be provided using an indication. The indication may be in any suitable format and may be rendered using for example visual and/or audio rendering. The indication may also, in some exemplary embodiments, be transmitted to another device, for example, to a user device from which the input data was received.

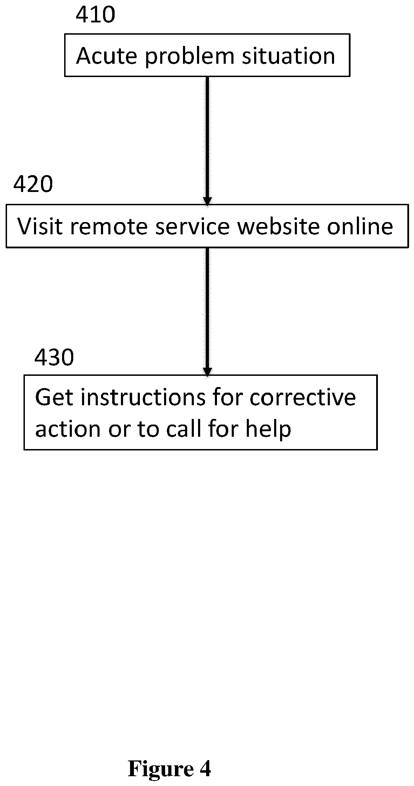

An exemplary embodiment of an environment extracting value from large amounts of data with the help of artificial intelligence and machine learning is illustrated in Figure 4. Initially there is an acute situation of problems or alarms 410. This may be for example a warning signal showing up in a factory machinery, an accident at home, or any unexpected situation exceeding the expertise of a user. The user visits a remote service website 420 by a network connection to feed input data of the situation by, for example, filling a questionnaire provided by the website or by selecting one or more options provided by the website. The input data may comprise data regarding the acute situation and it may comprise also some historical data provided by the user or a database the website service may have connection to. The website service provides recommendations 430 about how to tackle the situation. The website service may utilize a large database and machine learning algorithms to obtain likelihoods for groups of labels, based on which it provides the recommendations. The utilization may be for example such as described in the previous exemplary embodiments. The database may comprise data that may be utilized as a reference to determine the most recommended action for the given situation. Additionally, alternative actions may also be recommended as well as their determined likelihood of being successful.

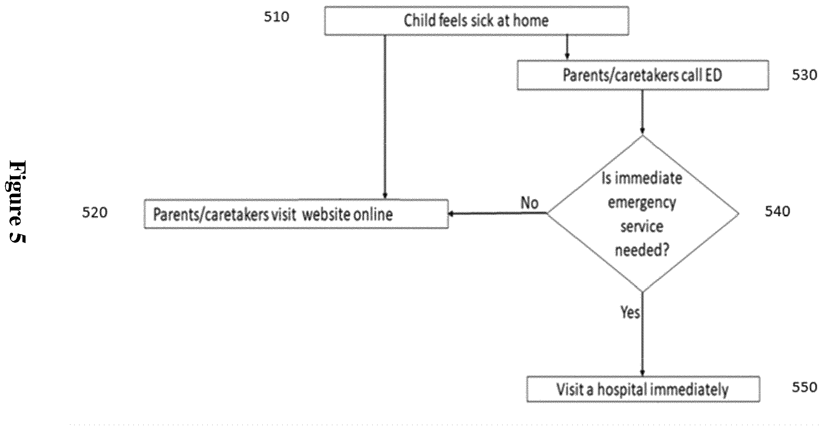

Figure 5 illustrates another exemplary embodiment of an environment extracting value from large amounts of data with the help of artificial intelligence and machine learning. Initially, in this exemplary embodiment, a child feels sick at home 510. A caretaker may contact a remote service website 520. This may be done for example with a user device such as the user device 110 introduced in Figure 1. The website 520 may be provided by a medical service provider and it may have access to machine learning algorithms trained on a large set of data such as those introduced in previous exemplary embodiments. The website may receive input data from the caretaker regarding the child's condition and then feed that input data to the machine learning algorithms. The website may also receive input data from a database regarding the child's historical healthcare data. The input data may be labelled by obtaining likelihoods for groups of labels by utilizing the machine learning algorithms, and then an indication may be rendered by the user device regarding the obtained labelling. The machine learning may be utilized for example according the previous exemplary embodiments. The indication may provide suggestions or further information regarding what the caretaker may wish to do and/or if it is recommended to seek for professional help by medical staff for example for diagnosing purposes. Alternatively, or additionally, the caretaker may call a hospital emergency department 530. The emergency department may check for need of immediate emergency service in the call 540, and either request an immediate visit to a hospital 550 or direct the caretaker to visit the remote service website 520. It is to be noted that although a website is mentioned here, any other suitable interface through which the machine learning algorithms may be utilized could be used additionally or alternatively.

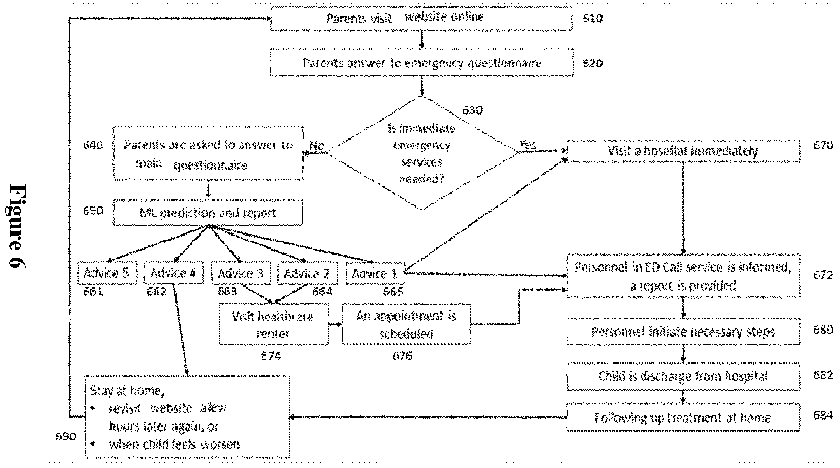

Figure 6 illustrates an exemplary embodiment of how the embodiment illustrated in Figure 5 may function. When parents or caretakers visit the website online 610, introduced in Figure 5 as 520, they are first asked to fill an emergency questionnaire 620, which may be executed by, for example, typing text, ticking boxes, adjusting sliders on a graphical user interface, recording a voice message, or some other means, to find out if an immediate visit to a hospital may be recommended 630. They may be instructed to visit a hospital immediately 670 by, for example, showing an instruction text, or offering a call connection to the hospital via the service website. Alternatively, they may be instructed to fill out a comprehensive questionnaire 640. This may be executed by, for example, typing text, ticking boxes, adjusting sliders on a graphical user interface, recording a voice message, or by some other means, to provide an input data to the service. The service may generate a machine learning prediction and a report 650 on the basis of the filled questionnaire. This may be done for example using a determination unit such as the determination unit 210 introduced in Figure 2. The results of the machine learning algorithms may be obtained as likelihoods for possible labels or groups of labels for the input data. The labels may comprise predictions for the child's health condition. The labelled input data may be utilized for deriving advice options 661, 662, 663, 664, 665, for the user regarding necessary measures and follow-up. The advice may comprise recommendations such as a visit to a hospital immediately 670 or within a certain time 674, for example, with a scheduled appointment 676, which may be reserved via the service website. A report comprising the machine learning prediction, advice, and/or the input data may be provided to the hospital personnel in advance 672 to be, for example, better prepared for a treatment and to save reception time. On the other hand, the caretakers may be recommended, based on the obtained labelled input data, to recontact the website, for example, within a certain time or if the child's condition gets worse 690. The recommendation may be provided as an indication. In case of a recommendation to visit a hospital, the child may be treated in hospital 680 and subsequently discharged 682. One aspect of follow-up treatment 684 may be to contact the website 690 to check if the child's condition needs re-hospitalization or not. The caretakers may be given a recommendation to contact the website, for example, after a certain time, in the event of certain symptoms, or whenever they have worries about the child's condition.

Another exemplary embodiment of a task ordinarily requiring human intelligence and executable by artificial intelligence is assessing a child's health condition. The task may be executed by a service provided remotely for a user in charge of a child. Input for the task may be provided by the user as data comprising present and historical health information of the child, and may be fed to the service by, for example, typing text, ticking boxes, adjusting sliders on a graphical user interface, recording a voice message, or by some other means. Additionally, input may be provided through an access to an external database comprising healthcare data. The input may be utilized as an input data or converted to an input data for machine learning algorithms. Likelihoods for possible labels for the input data may be obtained by one or more machine learning algorithms, and the likelihoods may then be utilized for labelling the input data. The utilization of the machine learning algorithms and the labelling may be for example such as described in the previous exemplary embodiments. An indication may be provided to the user regarding the labelled input data, for example such as illustrated in the previous exemplary embodiments. The indication may comprise advice or a recommendation regarding the child's care based on the labelling likelihoods obtained from the machine learning algorithms. The indication may comprise, for example, a recommendation to visit a hospital immediately, a recommendation to visit a hospital at a suggested appointment, a recommendation to stay at home but reconnect to the service in a predefined time, advice regarding medication or other care, or other advice or recommendation. The indication may be provided showing an instruction text and/or images, or offering a call connection to the hospital via the service website. The indication and the input data may also be provided to a hospital or another healthcare unit for being able to, for example, prepare for admitting the child as a patient or add the child's health data to an internal database.

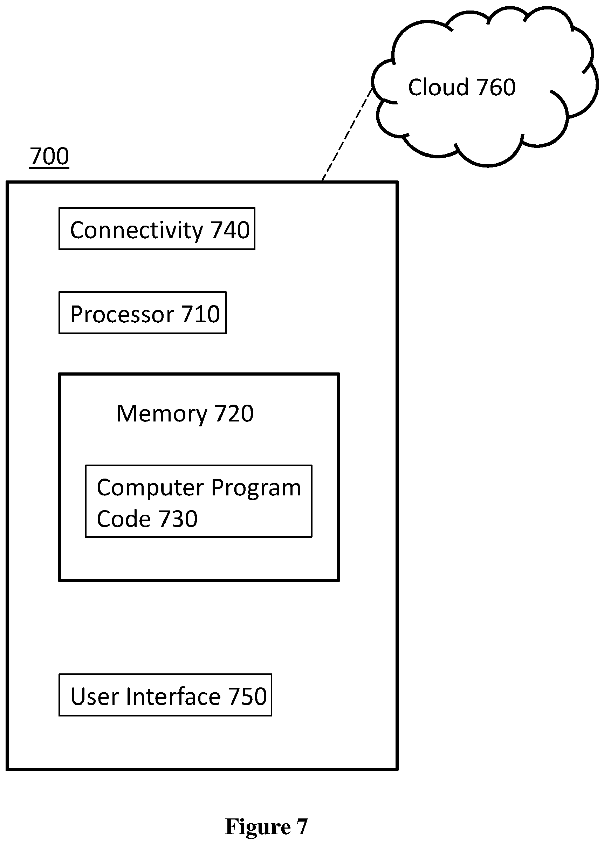

Figure 7 illustrates an exemplary embodiment of an apparatus such as device 110. The apparatus is applicable for performing one or more example embodiments of the invention. The apparatus 700 comprises a processor 710 that may comprise one or more processing cores containing circuitry configured to execute instructions comprised in computer program code 730. The computer program code 730 may be any such computer program code which can be stored, temporarily or permanently, in the memory 720 and executed by the processor 710. The memory 720 may be implemented using any suitable data storage technology, such as semiconductor-based memory devices, flash memory, magnetic memory devices and systems, optical memory devices and systems, fixed memory, and removable memory. The apparatus 700 may further comprise a connectivity unit 740 configured to enable wired and/or wireless connectivity. The wireless connectivity may be cellular connectivity, Wi-Fi connectivity, Bluetooth connectivity and/or any other suitable method of connectivity. A user interface 750 enables a user to interact with the apparatus 700. The user interface 750 may comprise for example a display, a loudspeaker, a keyboard, a mouse, a touch user interface, and/or a microphone. Any other suitable means for user interaction could also be comprised in the user interface 750.

## Claims

1. A method, comprising:
receiving input data from a network connection between two devices;
providing the input data to a determination unit;
labelling the input data using the determination unit, wherein the determination unit utilizes a plurality of Machine Learning algorithms that has been trained on a database, and wherein labelling comprises obtaining labelling likelihoods from a plurality of Machine Learning algorithms; and
providing an indication regarding the labelled input data.

2. A method according to claim 1, wherein the plurality of Machine Learning algorithms comprises at least one of: logistic regression, support vector machine, random forest, and naive bays.

3. A method according to claim 1 or 2, wherein the labelling of the input data is determined by calculating averages of the labelling likelihoods for groups of labels, the said labelling likelihoods provided by at least two of the plurality of Machine Learning algorithms.

4. A method according to claim 3, wherein a group of labels comprises at least two labels.

5. A method according to claim 3, wherein a group of labels comprises one label.

6. A method according to any of the claims 1-5, wherein the indication comprises a label that is determined, based on the labelling, to have the highest likelihood.

7. A method according to any of the claims 1-6, wherein the network connection is an internet connection.

8. A method according to any of the claims 1-7, wherein the input data comprises a filled questionnaire.

9. A method according to any of the claims 1-8, wherein the database has been prepared by one-hot encoding.

10. A method according to claim 9, wherein the input data is prepared by one-hot encoding.

11. A method according to any of the claims 1-10, wherein the labelled input data is added to the training database.

12. A method according to claim 11, wherein the plurality of Machine Learning algorithms is retrained with the added database.

13. A method according to any of the claims 1-12, wherein the input data comprises healthcare information and the indication comprises recommendation regarding a need for pediatric hospital emergency services.

14. An apparatus comprising means for performing a method according to any of the claims 1-13.

15. A computer program product comprising instructions for causing an apparatus to perform a method according to any of the claims 1-13.
